# EUROPÄISCHE PATENTANMELDUNG

(11) **EP 0 727 663 A1**
(43) Veröffentlichungstag der Anmeldung: **21.08.1996**
(21) Anmeldenummer: 96100604.6
(22) Anmeldetag: 17.01.1996
(51) Int. Cl.: G01N 33/543

(54) **Modifikation der Reaktivität von Agglutinationsreagenzien durch Cobeschichtung mit nicht gegen den Analyten gerichteten Substanzen**

(30) Priorität: 16.02.1995 DE 19505204
(71) Anmelder: BEHRINGWERKE AG, 35001 Marburg (DE)
(72) Erfinder: Kraus, Michael, Dr., D-35041 Marburg (DE)

(57) **Zusammenfassung**

Offenbart wird ein hyperkritisches Agglutinationsreagenzes zur Bestimmung eines Analyten umfassend ein Trägerteilchen, an das mindestens eine Art von gegen den Analyten gerichteter, affiner Bindungspartner und mindestens eine weitere Art von Liganden, die keine Affinität zum Analyten aufweisen, befestigt sind.

## Beschreibung

Die Erfindung betrifft ein hyperkritisches Agglutinationsreagenz umfassend Partikel, die mit sowohl gegen einen Analyten gerichteten Rezeptor als auch mit einer mit dem Analyten nicht reagierenden Substanz beschichtet sind.

Ein immunologisches Verfahren zur Bestimmung von Analyten beruht auf der Beschichtung von Partikeln mit Antikörpern gegen den gewünschten Analyten bzw. mit zu den gesuchten Antikörper-Analyten komplementären Antigenen (z.B. IgG-Beschichtung zum Nachweis von Rheumafaktoren) und dem anschließenden Nachweis der Immunreaktion. Als Partikel werden Proteine, Zellen (z.B. Erythrozyten oder Blutplättchen), Lipid- oder Gelatinevesikel, kolloidales Gold , Magnetpartikel und vor allem eine Vielzahl synthetischer Polymere in verschiedenen Größen und Modifikationen verwendet, die zur Optimierung der Beschichtung und der Nachweisreaktion entwickelt wurden. Die Nachweisreaktion, sofern sie nicht durch Augenschein verfolgbar ist, erfolgt mittels der Messung der Lichtstreuung (nephelometrisch), der entstehenden Trübung (turbidimetrisch) oder mittels Fluoreszenz-Dämpfung Auch die Bestimmung der Partikelgrößen wird angewandt. Diese prinzipiellen Verfahren sind dem Fachmann an sich hinreichend bekannt. Daneben sind jedoch oft weitere Modifikationen des Testansatzes erforderlich um einen möglichst störungsfreien und möglichst breiten Meßbereich für den gewünschten Analyten zu erreichen.

So ist ein Verfahren beschrieben, bei dem Proteine der Reaktionslösung zugesetzt werden, die mit dem in dieser Lösung nachzuweisenden Analyten keine Immunreaktion eingehen, sondern dazu dienen, unspezifische Kreuzreaktionen von Serumkomponenten, wie etwa durch C1q und Rheumafaktoren zu verhindern. Zum gleichen Zweck wird in einer anderen Publikation beispielsweise Pepsin der Reaktionslösung hinzugefügt.

Bekannt ist auch der Zusatz von Polyanionen zur Reaktionslösung zur Beschleunigung der Agglutinationsreaktion oder von Poly-Anethansulfonsäure zur Partikelsuspension zur Stabilisierung des Reagenzes.

Neben Zusätzen zur Reaktionslösung sind auch Verfahren beschrieben, in denen Partikel mit Proteinen beschichtet werden, die keine Bindungspartner des gesuchten Analyten sind. So kann in einem ersten Schritt die zu analysierende Lösung zunächst mit derartigen Partikeln in Kontakt gebracht werden, bevor in einem zweiten Schritt die eigentliche Agglutinationsreaktion mit gegen den Analyten gerichteten Partikeln verfolgt wird. Durch Differenzbildung aus beiden Messungen werden Störungen durch unspezifische Agglutinationsreaktionen ausgeblendet. Ähnliche Verfahren zur Unterdrückung von Störreaktionen sind auch in anderen Publikationen beschrieben.

In DD 0 282 529 wurden anti-CRP-Antikörper gleichzeitig mit anderen Proteinen wie Serumalbumin oder Glycoproteinen gemischt und zur Beschichtung verwendet. Dieses Verfahren diente zur Verringerung unspezifischer Agglutinationsreaktionen und zur besseren Dispergierbarkeit des anti-CRP-Reagenzes.

Bei der Entwicklung eines Reagenzes zum nephelometrischen Nachweis von D-Dimer, einem quervernetzten Abbauprodukt von Gerinnseln im Plasma, stellte sich das Problem, daß nach dem Verfahren von Kapmeyer et al. (EP 0 080 614) mit Antikörpern gegen D-Dimer beschichtete Latexpartikel eine so starke Dynamik der Agglutinationsreaktion mit dem Antigen (D-Dimer) aufwiesen, daß innerhalb von 4 geometrischen Verdünnungsstufen der Meßbereich des Detektors erschöpft war. Die Aufgabenstellung der Erfindung ist es, ein Agglutinationsreagenz zur Bestimmung eines Analyten zur Verfügung zu stellen, welches bei der Bestimmung des Analyten in der Lage ist, die Dynamik der Agglutinationsreaktion zu dämpfen, trotz der Dämpfung aber die analytische Sensitivität zu bewahren, wenn nicht sogar zu steigern.

Überraschenderweise wurde gefunden, daß bei der hyperkritischer Beschichtung eines Trägers in einem Gemisch aus einem gegen den Analyten gerichteten affinen Bindungspartner und einem nicht gegen den Analyten gerichteten Liganden, die Reaktionsdynamik des resultierenden Reagenzes beeinflußt werden kann. Die Reaktionskenetik war beschleunigt und beeinflußte dadurch sowohl die Signalausbeute als auch die Sensibilität positiv. Ein Reagenz, das eine solche Reaktion zeigt, kann mal als "hyperkritisch" bezeichnen.

Gegenstand der Erfindung ist daher ein Agglutinationsreagenz zur Bestimmung eines Analyten umfassend ein Trägerteilchen, an das mindestens eine Art von gegen den Analyten gerichteter, affiner Bindungspartner und mindestens eine weitere Art von Liganden, die keine Affinität zum Analyten aufweisen, befestigt sind, wobei das Agglutinationsreagenz im hyperkritischen Zustand vorliegt.

Der hyperkritische Zustand des Agglutinationsreagenzes wird sowohl durch die Beschichtungskonzentration der jeweiligen Beschichtungspartner, insbesondere über Auswahl und Konzentration des nicht-affinen Liganden, als auch durch die Gesamtkonzentration aller Beschichtungspartner (d. h. affine Bindungspartner und nicht-affine Liganden) gesteuert. Die Gesamtkonzentration der Beschichtungspartner des erfindungsgemäßen Agglutinationsreagenzes liegt dabei knapp unterhalb des Sättigungswertes der Beschichtungskapazität des Trägermaterials, d. h. bei 50 - 95 % der theoretisch möglichen maximalen Beschichtung. Der Anteil des nicht-affinen Liganden an der Gesamtkonzentration aller Beschichtungspartner beträgt 10 - 75 mol-%, insbesondere 20 - 60 mol-%. Das erfindungsgemäße Agglutinationsreagenz steht dabei kurz vor der Eigenagglutination.

Als feste, wasserunlösliche Träger sind dem Fachmann an sich bekannte, natürliche und synthetische, organisch und anorganische Polymere geeignet, wie beispielsweise: Polystyrol, Polydextrane, Plypropylen, Polyvinylchlorid, Poyvinylidienfluorid, Poylacrylamid, Agarose, Latex, Magnetit, poröses Glaspulver, Erytrhozyten, Leukozyten, Blutplättchen oder Copolymere aus Styrol-Butadien, Styrol-Methacrylsäure oder Methacrylat-Methacrylsäure, aber auch Goldpartikel oder Vesikel aus Lipiden oder Gelatine. Der bevorzugte Träger ist Latex.

Als affiner Bindungspartner können einer oder mehrere Antikörper, Antigene, Lectine, Avidin, Streptavidin oder andere Substanzen eingesetzt werden, die spezifisch an den gesuchten Analyten binden, wobei Antikörper und Antigene, insbesondere monoklonale Anktikörper, bevorzugt sind.

Als reaktionsmodifizierende nicht-affine Liganden können zur Beschichtung einzelne oder mehrere Proteine verwendet werden. Neben natürlicherweise vorkommenden Proteinen können auch derivatisierte Proteine oder gentechnisch bzw. synthetisch hergestellte Proteine oder Peptide verwendet werden. Bevorzugterweise werden dabei als nicht-affine Liganden Antikörper aus der gelcieh Spezies (Tierart) vewendet wie die` der affinden Bindungspartner. Als synthetische Peptide werden bevorzugterweise solche Peptide verwendet, deren Nettoladung unter Testbedingungen entgegengesetzt zur der Nettoladung der zum Test verwendeten Festphase ist.

Das erfindungsgemäße Agglutinationsreagenz ist um den Faktor 10 bis 100 empfindlicher als herkömmliche Agglutinationsreagenzien. Diese Steigerung der Empfindlichkeit ist dabei nicht auf eine Reaktion mit dem Antigen zurückzuführen, sondern ist vielmehr auf den hyperkritischen Zustand mit der Neigung zur Eigenagglutination der beschichteten Partikel zurückzuführen, denn es waren insbesondere solche Proteinen geeignet, die bei alleiniger Beschichtung im Test eine höheres Meßsignal aufwiesen. Dieses Verfahren steht daher im genauen Gegensatz zu der in DD 282 529 beschriebenen Absicht, ein möglichst nicht selbst aggregierendes Reagenz herzustellen. Die Ursache für den beobachteten Effekt ist wahrscheinlich die Neutralisierung der Eigenladung der Partikel, die diese normalerweise in der Schwebe hält. Daher können zur Beschichtung zusammen mit den affinen Bindungspartnern auch synthetische Peptiden, wie etwa poly-Anionen oder poly-Kationen, verwendet werden, die die Nettoladung der Partikel neutralisieren. Die erfindungsgemäßen Agglutinationsreagenziehn zeichnen sich darüberhinaus durch eine bessere Haltbarkeit und Stabilität aus.

Gegenstand der Erfindung ist weiterhin ein Verfahren zur Herstellung des vorstehend beschriebenen Agglutinationsreagenzes, wobei die affinen Bindungspartner und die nicht-affinen Liganden in einem oder mehreren Schritten an das Trägerteilchen gekoppelt werden.

Das erfindungsgemäße Agglutinationsreagenz kann dabei nach dem Fachmann an sich bekannten Kopplungsverfahren hergestellt werden (z. B. EP 0 080 614). So können die Proteine über ihre Aminofunktion an Aldehydgruppen einer Schale um Polystyrolpartikel mittels der Schiff'schen Base Reaktion gekoppelt und durch Reduktion mit Borhydrid irreversibel immobilisiert werden.

Dieses Verfahren zur Beschichtung der Partikel ist eine Modifikation bisheriger Verfahren, die die eigentlichen Immunreaktion nicht betrifft und daher auf alle dem Fachmann an sich bekannten Verfahren zur Beschichtung und Verwendung von Partikeln angewandt werden kann.

Gegenstand der Erfindung sind auch diagnostische Verfahren zur immunchemischen Bestimmung eines Analyten unter Verwendung des vorstehend beschriebenen Agglutinationsreagenzes durch Messung mittels Augenschein, nephelometrisch, turbidimetrisch, mittels Fluoreszenz-Dämpfung oder durch Bestimmung der Größen der entstehenden Immunkomplexe.

In einer bevorzugten Ausführungsform wird mit diesem Verfahren humanes D-Dimer bestimmt, wobei der affine Bindungspartner der Antikörper DD5 (DSM ACC2201) ist und/oder der nicht-affine Ligand der Antikörper NTIDYO (DSM ACC2202) ist.

Gegenstand der Erfindung ist außerdem die Verwendung der erfindungsgemäßen Partikel zur therapeutischen Zwecken, insbesondere dann, wenn dieses gegen Abwehrmechanismen im Körper geschützt sein muß. Dieser Schutz gegen Abwehrmechanismen umfaßt dabei sowohl Schutz gegen zelluläre (z. B. phagocytäre) als auch humorale Systeme (proteolytischen Abbau).

Gegenstand der Erfindung ist ferner die Verwendung des Agglutinationsreagenzes zur Immunisierung von Tieren oder Menschen und zur Verlängerung der Halbwertszeit von im Agglutinationsreagenz eingeschlossenen Wirksubstanzen im Körper.

Die vorliegende Erfindung wird durch die Abbildungen näher erläutert.
**Figur 1:**
   Reaktion von nur mit dem spezifischen Antikörper DD5 (--·--) bzw. mit einer Mischung aus spezifischen und nicht gegen den Analyten gerichteten Antikörper NTIDYO (DD5/NTIDYO, -··△··-) beschichteten Latexpartikeln mit gereinigtem, humanen D-Dimer. AU = relatives Meßsignal am Nephelometer.
**Figur 2:**
   Reaktion von nur mit dem spezifischen Antikörper DD5 (--·--) bzw. mit einer Mischung aus spezifischen und nicht gegen den Analyten gerichteten Antikörper PNOB (DD5/PNOB; -··△··- ) beschichteten Latexpartikeln mit gereinigtem, humanen D-Dimer. AU = relatives Meßsignal am Nephelometer.
**Figur 3:**
   Reaktion von nur mit dem spezifischen Antikörper DD5 (--·--) bzw. mit einer Mischung aus spezifischen und nicht gegen den Analyten gerichteten Antikörper PHTN (DD5/PHTN, -··△··-) beschichteten Latexpartikeln mit gereinigtem, humanen D-Dimer. AU = relatives Meßsignal am Nephelometer.
**Figur 4:**
   Reaktion von nur mit dem spezifischen Antikörper DD5 (--·--) bzw. mit einer Mischung aus spezifischen und nicht gegen den Analyten gerichteten Antikörper SUPRN (DD5/SUPRN; -··△··-) beschichteten Latexpartikeln mit gereinigtem, humanen D-Dimer. AU = relatives Meßsignal am Nephelometer.
**Figur 5:**
   Reaktion von nur mit dem spezifischen Antikörper DD5 (--·--) bzw. mit einer Mischung aus spezifischen Antikörper DD5 und poly-Lysin (DD5/poly-Lys; -··△··-) beschichteten Latexpartikeln mit gereinigtem, humanen D-Dimer. AU = relatives Meßsignal am Nephelometer.

Besonders bevorzugte Ausführungsformen sind den nachfolgenden, die Erfindung erläuternden, sie jedoch nicht einschränkenden Beispielen zu entnehmen.

### Beispiel 1

### Herstellung von anti-human-D-Dimer Latexreagenzien nach einem herkömmlichen Verfahren

Die Herstellung von Latexreagenzien erfolgte nach Kapmeyer W.H. et al., J. Clin. Lab. Anal. 2: 76-83 (1988). 1 ml eines Propfpolymerisats (4 %-ige Lösung; Fa. Behringwerke AG) wurde mit 0,1 ml des gegen D-Dimer spezifischen, monoklonalen Antikörper (Bezeichnung: DD5; Behringwerke AG; Konzentration: 0,5 mg/ml) und 0,05 ml einer 20 %-igen wäßrigen Tween® 20 Lösung gemischt. Zur Aktivierung der geschützten Aldehydgruppen auf dem Schalenpolymerisat wurde die Suspension mit ca. 0,01 ml einer 1 N HCl-Lösung auf einen pH von 2,5 eingestellt. Nach einer 30minütigen Inkubation bei Raumtemperatur wurden 0,25 ml einer gesättigten Natriumhydrogenphosphat-Lösung (pH 6,5) und 0,25 ml einer wäßrigen Natriumborhydrid-Lösung (25 mg/ml) hinzugefügt und intensiv gemischt. Die Kopplung des Antikörpers an die aktiverten Aldehydgruppen erfolgte über 1 Stunde bei Raumtemperatur. Anschließend wurde das Latex-Antikörper Konjugat zentrifugiert (Beckman Zentrifuge, 40000xg, 30 Minuten) und das Pellet in 1,5 ml eines 0,1 molaren Glycin-Puffers (pH 8,2; enthaltend 0,17 M NaCl und 0,5 % Tween® 20) resuspendiert. Die Lösung wurde mit Ultraschall für ca. 5 Sekunden behandelt (Bronson Sonifier B 15). Diese Stammlösung wurde bei +4 °C gelagert. Die Reaktion dieses Reagenzes mit humanem D-Dimer ist in Tabelle 1 aufgeführt.

### Beispiel 2

### Durchführung nephelometrischer Bestimmungen

Die Reaktion der anti-human-D-Dimer Latexreagenzien mit humanem D-Dimer (Behringwerke AG) wurde am Nephelometer BNA (Fa. Behringwerke AG, Marburg) verfolgt. Die wie unter Beispiel 1 hergestellten Stammlösungen wurden mit physiologischer Kochsalzlösung auf eine Konzentration von 0,03 % (w/v) verdünnt. 50 µl dieser Suspension wurden mit 20 µl D-Dimer Zusatzreagenz (Fa. Behringwerke AG) und 80 µl N-Diluens (Fa. Behringwerke AG) gemischt. Nach Zugabe von 50 µl Probe und 70 µl N-Diluens wurde die Differenz aus der Trübungzunahme nach 10 Sekunden und nach 12minütiger Inkubation bestimmt.

### Beispiel 3

### Herstellung von anti-human-D-Dimer Reagenzien nach dem erfindungsgemäßen Verfahren.

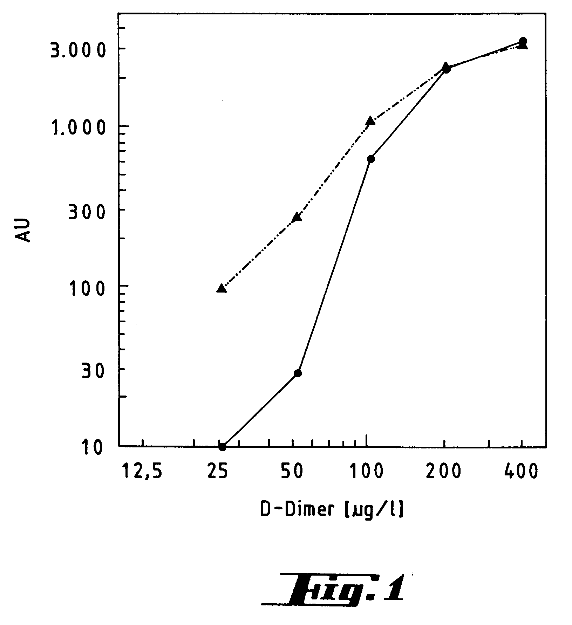

Das unter Beispiel 1 aufgeführte Verfahren wurde wie folgt verändert. Anstelle der 0,1 ml Antikörperlösung des Klons DD5 wurde ein Gemisch des anti-D-Dimer spezifischen Klons DD5 mit einem anti-human-IgE-Antiideotyp Antikörper (Bezeichnung: NTIDYO 80/63; Fa. Behringwerke AG) zur Beschichtung verwendet. Die Konzentration der Antikörper betrug jeweils 0,5 mg/ml. Der Einfluß auf die Reaktivität des resultierenden Latexreagenzes ist vergleichend zu dem nach herkömmlichen Verfahren nach Beispiel 1 hergestellten anti-D-Dimer Reagenz in Tabelle 1 und in Abbildung 1 aufgeführt. Es ist deutlich zu erkennen, daß das erfindungsgemäß hergestellten anti-human-D-Dimer Reagenz ein höhere Sensitivität und einen weiteren Meßbereich aufweist, als das parallel mit den gleichen Ausgangsmaterialien nach herkömmlichen Verfahren hergestellte Reagenz.

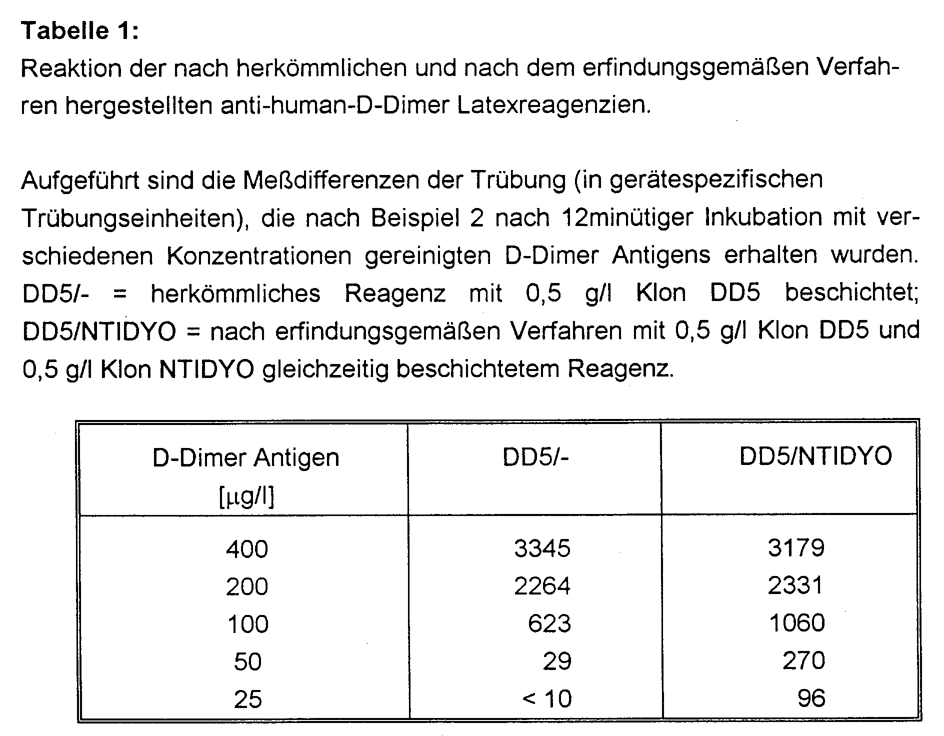

### Beispiel 4

### Steuerung des Agglutinationsverhaltens durch Variation der Konzentration von spezifischen und neutralen Beschichtungsantikörper

Nach dem unter Beispiel 3 beschriebenen Verfahren wurden Antikörper des Klons DD5 zusammen mit Antikörper des Klons NTIDYO 80/63 (beide Fa. Behringwerke AG) zur Beschichtung verwendet. Die Konzentration des spezifischen Antikörpers (DD5) betrug 0,5 mg/ml, die Konzentration des nicht mit dem Analyten (D-Dimer) reagierenden Antikörpers (NTIDYO) wurde von 0 - 1,0 mg/ml variiert. Der Einfluß auf die Reaktivität des resultierenden Latexreagenzes ist in Tabelle 2 aufgeführt. Es ist deutlich zu erkennen, daß die Sensitivität des erfindungsgemäß hergestellten anti-human-D-Dimer Reagenzes mit steigendem Anteil an neutralem Klon NTIDYO zunimmt. Bei hohen Konzentrationen kommt es jedoch wieder zu einer Verringerung des Signals, da dann der spezifische Antikörper von der Festphase verdrängt wird.

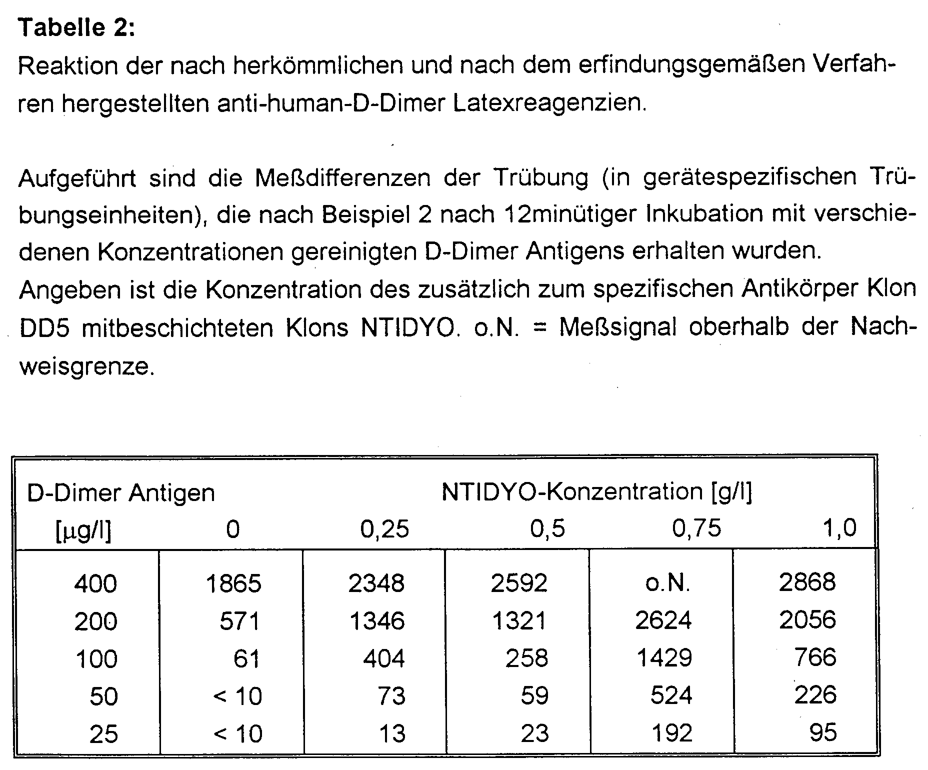

### Beispiel 5

### Eignung verschiedener Antikörper zur Modulation der Reaktionskinetik

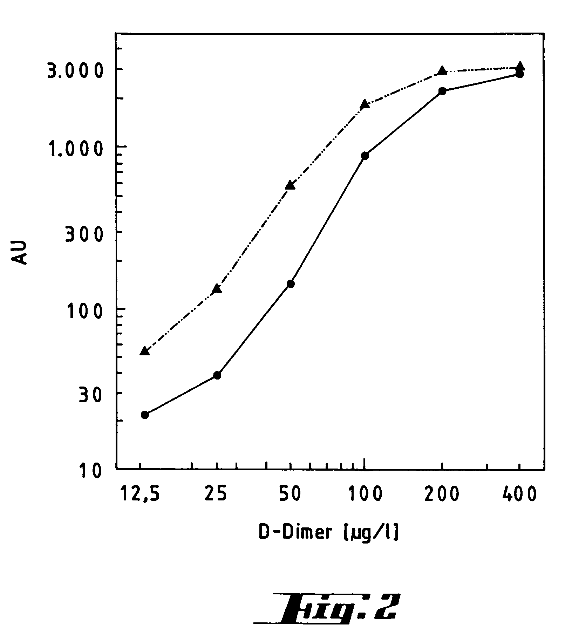

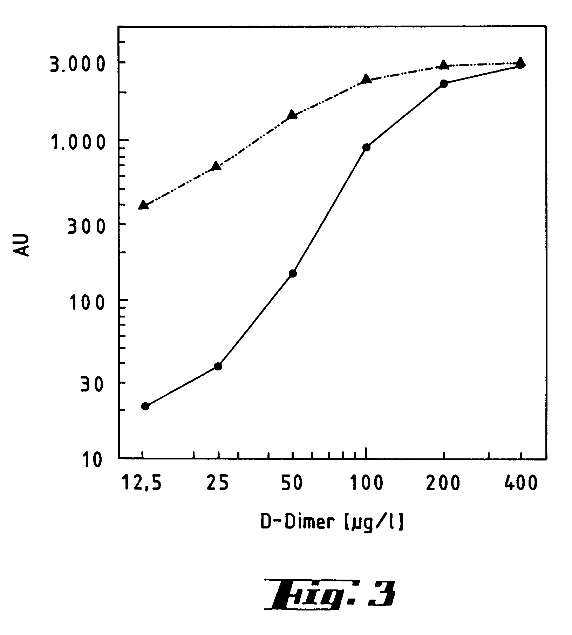

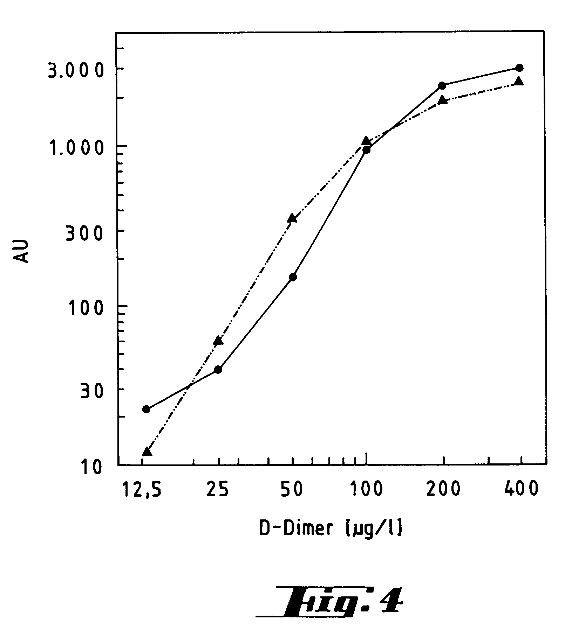

Nach dem in Beispiel 3 beschriebenen Verfahren wurden verschiedene weitere monoklonale Antikörper zur gleichzeitigen Beschichtung mit dem anti-human-D-Dimer Antikörper Klon DD5 verwendet, die nicht gegen den Analyten gerichtet sind und sinnvollerweise auch nicht mit anderen Antigenen in Körperflüssigkeiten reagieren, die zu einer Agglutinationsreaktion führen. So wurden zwei Klone gegen Haptene (Klon PNOB 87-28/10 gegen Phenobarbital, Klon PHTN 87-3/4 gegen Phenytoin), sowie ein weiterer anti-ideotypischer Antikörperklon (Klon SUPRN 87-3/25) verwendet. Die Beschichtungskonzentrationen betrugen jeweils 0,5 mg/ml. In Tabelle 3 und in den korrespondierenden Figuren 2 bis 4 sind die nach Beispiel 2 erhaltenen Meßsignale am BNA im Vergleich zu den Meßsignalen eines herkömmlich hergestellten Reagenzes (Beispiel 1) aufgeführt. Die gleichzeitige Beschichtung mit den Klonen PNOB und PHTN führte zu einer generellen Anhebung des Meßsignals, besonders aber im unteren Bereich und somit zu einer Verbesserung der Sensitivität und Ausweitung des Meßbereichs (besonders bei Verwendung des Klons PHTN). Antikörper des Klons SUPRN hingegen verringerten die Dynamik des Reagenzes besonders bei höheren Antigen Konzentrationen und erlauben daher die Erweiterung des Meßbereichs hin zu höheren Konzentrationen.

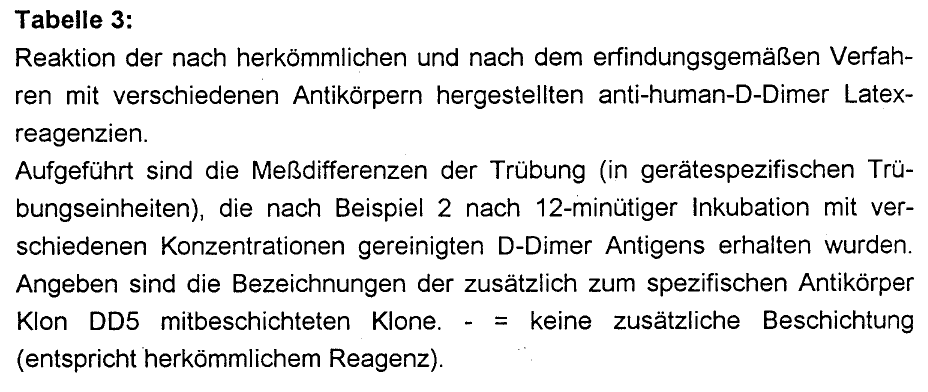

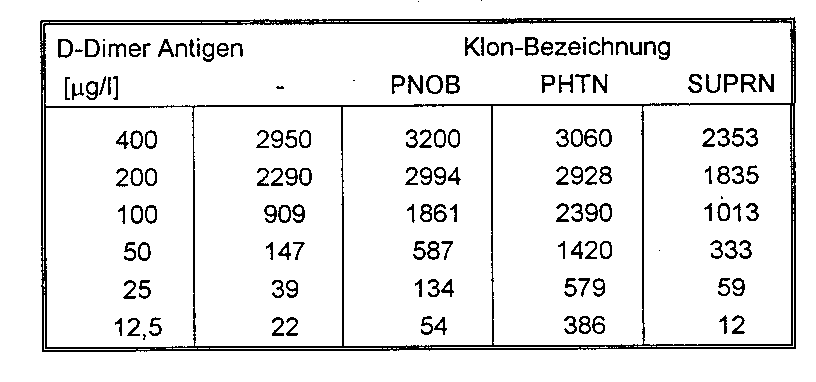

### Beispiel 6

### Reaktivität der zur Modulation verwendeten Proteine mit dem Analyten

Die in Beispiel 5 verwendeten Antikörper wurden nach dem herkömmlichen Verfahren in Konzentrationen von 0,5 mg/ml wie in Beispiel 1 auf den gleichen Ausgangsmaterialien wie unter Beispiel 5 beschichtet und die Agglutinationsreaktion mit D-Dimer Antigen überprüft. Wie aus Tabelle 4 hervorgeht, wurde kein von der Konzentration des Analyten abhängiges Signal erzeugt. Die Modulation der Aktivität wie in den Beispielen 3 bis 5 beschrieben hängt vielmehr mit der erhöhten Neigung zur Eigenaggregation der Partikel zusammen.

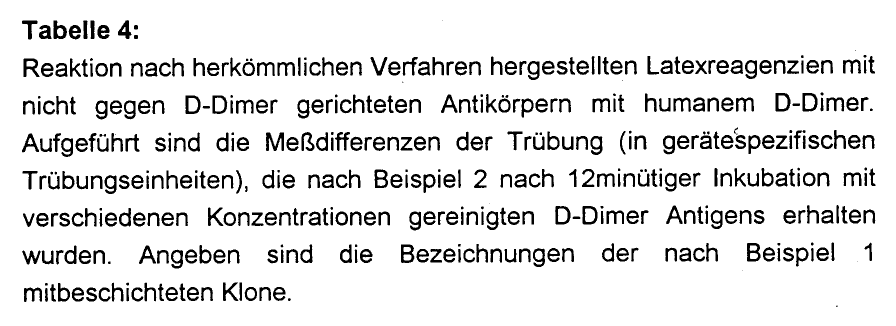

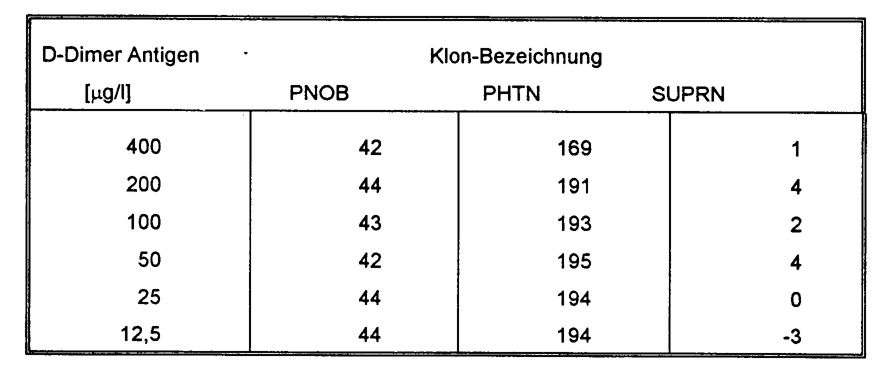

### Beispiel 7

### Anwendung des erfindungsgemäßen Verfahrens mit synthetischen Peptiden

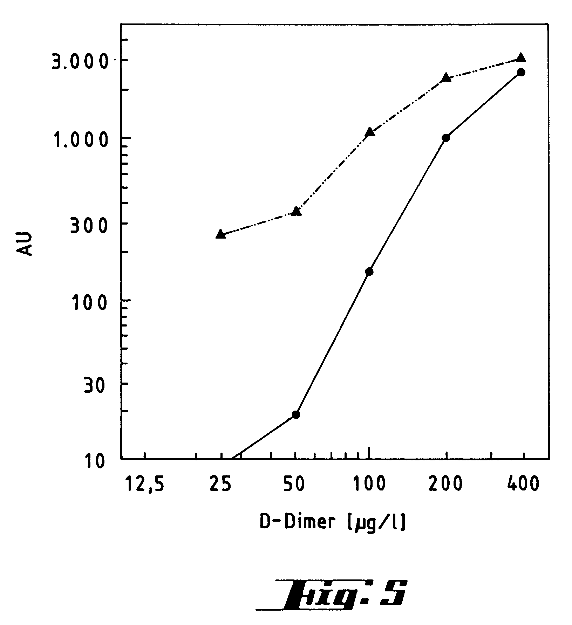

Analog zum unter Beispiel 3 aufgeführten Beschichtungsverfahren wurde anstelle von natürlichen Proteinen (hier: monoklonale Antikörper) eine Mischung des anti-human D-Dimer spezifischen Antikörperklons DD5 mit synthetischen Peptiden zur Beschichtung verwendet. Im Beispiel wurde ein Gemisch von 0,5 mg/ml Antikörper DD5 mit 1 mg/ml poly-DL-Lysin (Molekulargewicht: 15 bis 30 kD; Fa. Sigma, Deisenhofen) zur Beschichtung eingesetzt. In Tabelle 5 und Abbildung 5 ist die modulierende Wirkung auf die Bestimmung von humanem D-Dimer nach Beispiel 2 parallel zu nach herkömmlichen Verfahren (Beispiel 1) nur mit 0,5 mg/ml Antikörper DD5 beschichteten Latexpartikeln dargestellt. In diesem Beispiel wurde die Reaktionsdynamik insbesondere im unteren Bereich erhöht. Die gleichzeitige Beschichtung der Partikel mit spezifischem Antikörper und synthetischem Peptid führte somit ebenfalls zu einer Modulation des Agglutinationsverhaltens, die zur Steuerung der Sensitivität und des Meßbereichs von auf Partikel basierenden Reagenzien verwendet werden kann. Als synthetisches Peptid wurde in diesem Beispiel ein poly-Kation verwendet, da die zur Beschichtung verwendeten Partikel auf Grund der zur Synthese der Schale verwendeten Methacrylsäure eine negative Nettoladung besitzen und durch die Beschichtung diese Ladung verringert werden sollte.

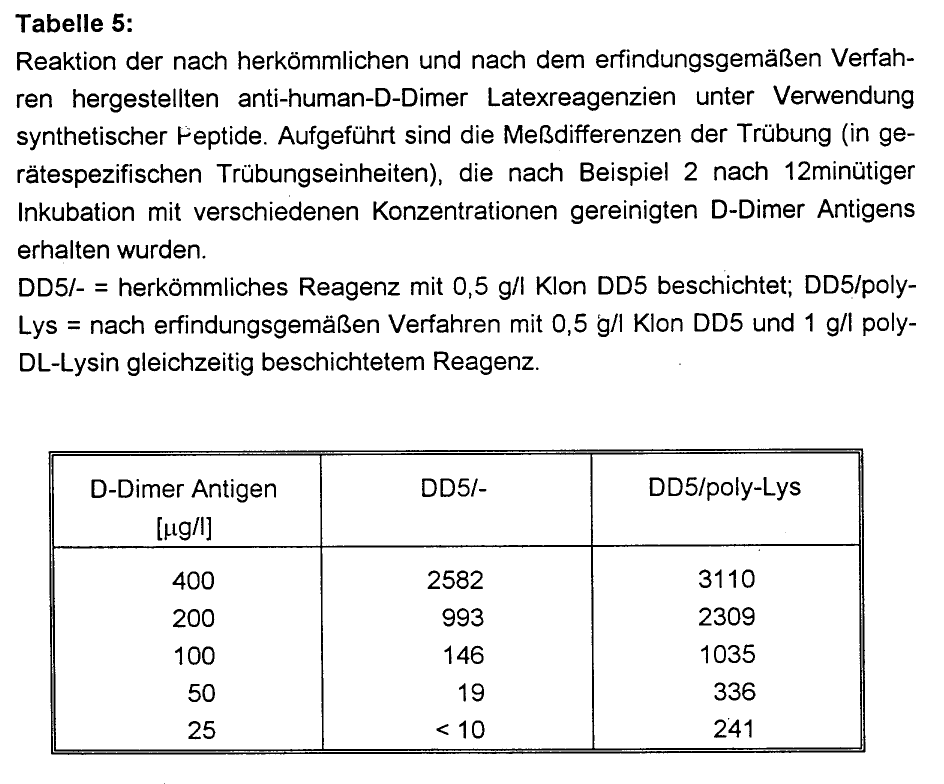

Die Hybridomzellen FBN FRG DD5 und NTIDYO 80/63 wurden am 08. Dezember 1994 bei der DSM - Deutsche Sammlung von Mikroorganismen und Zellkulturen GmbH, Maschroder Weg 1b, D-38124 Braunschweig - unter der Bezeichnung DSM ACC 2201 und DSM ACC 2202 hinterlegt.

## Patentansprüche

1. Agglutinationsreagenz zur Bestimmung eines Analyten umfassend ein Trägerteilchen, an das mindestens eine Art von gegen den Analyten gerichteter, affiner Bindungspartner und mindestens eine weitere Art von Liganden, die keine Affinität zum Analyten aufweisen, befestigt sind, dadurch gekennzeichnet, daß das Agglutinationsreagenz im hyperkritischen Zustand vorliegt.

2. Agglutinationsreagenz nach Anspruch 1, dadurch gekennzeichnet, daß das Trägerteilchen ein festes, wasserlösliches Material ist, insbesondere ein organisches oder anorganisches Polymer, wie Polystyrol, Polydextran, Polypropylen, Polyvinylchlorid, Polyvinylidienfluorid, Polyacrylamid, Agarose, Latex, Magnetit oder poröses Glaspulver, Copolymer aus Styrol-Butadien, Styrol Methacrylsäure oder Methacrylat-Methacrylsäure, aber auch Goldpartikel oder Vesikel aus Lipiden, Erythrozyten, Leukozyten, Blutplättchen und Gelatine.

3. Agglutinationsreagenz nach Anspruch 2, dadurch gekennzeichnet, daß die Trägerteilchen aus Latex sind.

4. Agglutinationsreagenz nach irgendeinem der Ansprüche 1-3, dadurch gekennzeichnet, daß der affine Bindungspartner ausgewählt ist aus einem oder mehreren Antikörpern, Antigenen, Lectinen, Avidin, Streptavidin und anderen Substanzen, die spezifisch an den zu bestimmenden Analyten binden.

5. Agglutinationsreagenz nach Anspruch 4, dadurch gekennzeichnet, daß der affine Bindungspartner ein Antigen oder ein Antikörper, insbesondere ein monoklonaler Antikörper ist.

6. Agglutinationsreagenz nach irgendeinem der Ansprüche 1-5, dadurch gekennzeichnet, daß die nicht-affinen Liganden natürlich vorkommende, derivatisierte oder synthetisch hergestellte Proteine, insbesondere Antikörper sind.

7. Verfahren zur Herstellung eines Agglutinationsreagenzes nach irgendeinem der Ansprüche 1-6, dadurch gekennzeichnet, daß die affinen Bindungspartner und die nicht-affinen Liganden in einem oder mehreren Schritten an das Trägerteilchen gekoppelt werden.

8. Diagnostisches Verfahren zur immunchemischen Bestimmung eines Analyten unter Verwendung des Agglutinationsreagenzes gemäß irgendeinem der Ansprüche 1-6.

9. Verfahren nach Anspruch 8 umfassend die Messung der Agglutinationsreaktion mittels Augenschein, nephelometrisch, turbidimetrisch, mittels Fluroeszenz-Dämpfung oder durch Bestimmung der Größe des entstehenden Immunkomplexes.

10. Verfahren nach Anspruch 8 oder 9 dadurch gekennzeichnet, daß der zu bestimmende Analyt humanes D-Dimer ist.

11. Verfahren nach Anspruch 10, dadurch gekennzeichnet, daß der affine Bindungspartner der Antikörper DD5 (DSM ACC 2201) ist und/oder der nicht-affine Ligand der Antikörper NTIDYO (DSM ACC 2202) ist.

12. Verwendung des Agglutinationsreagenzes gemäß irgendeinem der Ansprüche 1-6 zu therapeutschen Zwecken.

13. Verwendung des Agglutinationsreagenzes gemäß irgendeinem der Ansprüche 1-6 zu Immunisierung von Tieren und Menschen.

14. Verwendung des Agglutinationsreagenzes gemäß irgendeinem der Ansprüche 1 bis 6 zur Verlängerung der Halbwertszeit von in Partikeln eingeschlossenen Wirksubstanzen im Körper.
